# FASCICULE DE BREVET EUROPEEN

(11) **EP 0 490 714 B1**
(45) Date de publication et mention de la délivrance du brevet: **15.05.1996**
(21) Numéro de dépôt: 91402967.3
(22) Date de dépôt: 06.11.1991
(51) Int. Cl.: A61B 17/02, A61B 19/00

(54) **Instrument chirurgical endoscopique pour le déplacement de tissus ou organes**
Endoskopisches chirurgisches Instrument zum Manipulieren von Geweben oder Organen
Endoscopic surgical instrument for manipulating tissue or organs

(30) Priorité: 06.11.1990 FR 9013711
(43) Date de publication de la demande: 17.06.1992
(73) Titulaire: ETHICON INC., Somerville New Jersey 08876 (US)
(72) Inventeur: Bilweis, Joseph, 78560 Noisy Le Roy (FR)
(74) Mandataire: Martin, Jean-Jacques

(56) Documents cités:
- GB-A- 2 071 502
- US-A- 3 831 587
- US-A- 4 190 042
- US-A- 4 744 363

## Description

La présente invention concerne le domaine des instruments chirurgicaux.

La présente invention vise en particulier le domaine de la chirurgie endoscopique.

Les chirurgiens savent que les instruments jusqu'ici proposés pour saisir et/ou déplacer des tissus et/ou organes, notamment lors d'interventions endoscopiques, ne donnent pas totalement satisfaction.

Ces instruments sont généralement formés de pinces ou équivalents et occasionnent fréquemment des traumatismes sur les tissus ou organes déplacés.

La présente invention a pour but d'éliminer les inconvénients des instruments antérieurs connus.

Le document US-A-3831587 décrit un dispositif conforme au préambule de la revendication 1 annexée, qui comporte un support tubulaire parcouru par une canalisation interne connectée à l'une de ses extrémités à une source de pression et à l'autre extrémité à une structure annulaire gonflable. Les préoccupations du document US-A-3831587 sont tout-à-fait différentes de celles de l'invention puisqu'il concerne le domaine de l'insémination artificielle.

Le document US-A-4744363 décrit un instrument chirurgical pour déplacer des tissus comportant deux tiges sollicitées en écartement par un ressort et supportant une membrane souple.

Ce but est atteint selon la présente invention grâce à un instrument chirurgical, notamment pour chirurgie endoscopique, du type défini en revendication 1 annexée.

D'autres caractéristiques, buts et avantages de la présente invention apparaîtront à la lecture de la description détaillée qui va suivre, et en regard des dessins annexés donnés a titre d'exemples non limitatifs et sur lesquels :
- la figure 1 représente une vue schématique d'un dispositif conforme à la présente invention, en position de repos rétractée dans une canule,
- la figure 2 représente une vue du même dispositif en position de travail déployée, et
- la figure 3 représente une vue schématique en coupe longitudinale de l'instrument comprenant le tube, la poche en forme de palette et la poire de gonflage/dégonflage.

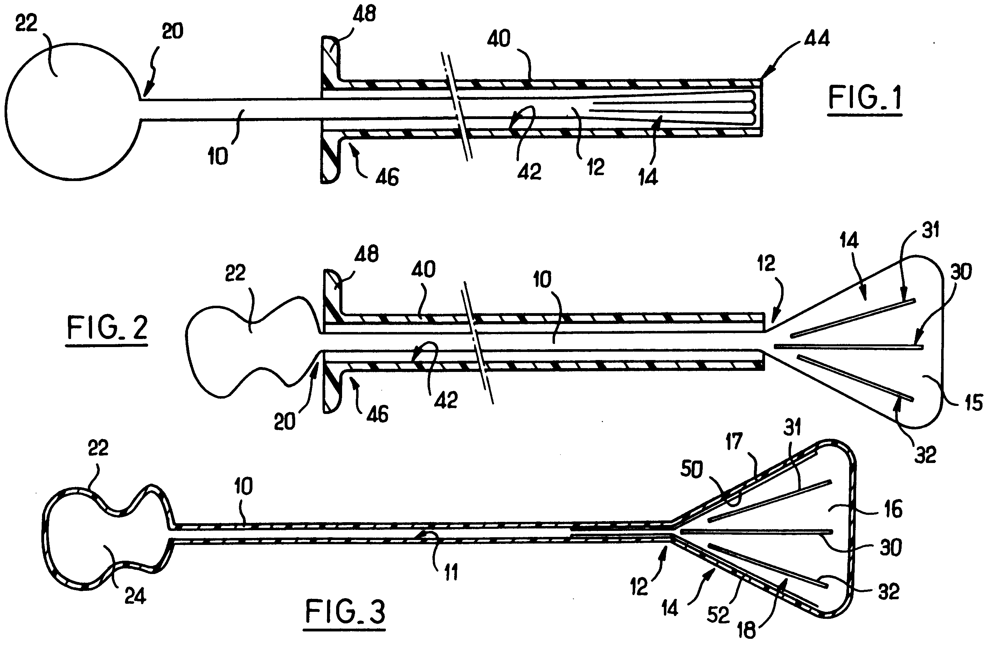

On aperçoit sur les figures 1 à 3 annexées un instrument apte à déplacer des tissus ou organes d'un être vivant et qui comprend essentiellement un tube 10 muni à son extrémité proximale 12 d'une poche gonflable 14, et muni à son extrémité distale 20 d'une poire 22.

La représentation donnée sur les figures annexées n'est bien entendu que schématique. En particulier, les dimensions relatives du tube 10, de la poche gonfable 14, et de la poire 22, représentés schématiquement sur les figures annexées, ne sont aucunement limitatives.

La poche gonflable 14 à l'état gonflé à la forme générale d'un éventail. C'est-à-dire que la poche 14 est sensiblement plane. Vue en plan, comme représenté sur les figures 2 et 3, la poche gonflable 14, à l'état gonflé, présente une forme générale triangulaire ou en secteur de cylindre.

La poche 14 est ainsi formée de deux toiles principales 15, 16, visibles sur les figures 2 et 3, généralement parallèles entre elles à l'état gonflé, et raccordées sur leur périphérie par une paroi de liaison transversale 17. Les toiles principales 15, 16 sont parallèles au plan des figures 2 et 3. La paroi de liaison périphérique 17 s'étend dans une direction générale transversale au plan des figures 2 et 3.

De préférence, la poche gonflable 14 formant palette est munie de membrures ou nervures longitudinales. Celles-ci sont représentées schématiquement sous les références 30, 31, 32 sur les figures 2 et 3.

On a ainsi représenté trois membrures ou nervures de raidissement sur ces figures. Ce nombre de membrures ou nervures de raidissement n'est cependant pas limitatif.

Les membrures ou nervures de raidissement 30, 31, 32 convergent vers la zone de liaison de la poche gonflable 14 sur l'extrémité distale 12 du tube 10.

Le volume interne 18 de la poche gonflable 17 communique avec le volume interne 24 de la poire de gonflage 22 par l'intermédiaire de la lumière interne 11 du tube 10. Le tube 10, la poche gonflable 14 et la poire 22 forme un système fermé étanche.

De préférence le tube 10, la poche gonflable 14 et la poire 22 sont réalisés sous forme d'une pièce unique. Cependant, le tube 10, la poche gonflable 14 et la poire 22 peuvent être réalisés sous forme de pièces initialement séparées, puis assemblées à l'aide de toute technique connue de l'homme de l'art.

Le matériau composant la poire 22 doit être un matériau souple élastique pour permettre à celle-ci de recouvrir sa position de repos expansée, comme représenté sur la figure 1 après sollicitation. De préférence, le matériau composant le tube 10, la poche gonflable 14 et la poire 22 est du caoutchouc. En variante, des moyens de rappel élastiques peuvent être prévus à l'intérieur de la poire 22.

Les membrures ou nervures de raidissement 30, 31, 32 peuvent être réalisées dans le même matériau de base que la poche gonflable 14, sous forme d'épaississements localisés de la paroi de celle-ci. En variante, les membrures ou nervures de raidissement 30, 31, 32 peuvent être rapportées sur la poche 14.

De façon avantageuse, comme cela est représenté schématiquement sur les figures annexées, le tube 10 est placé dans la lumière 42 d'une canule 40. Cette canule 40 peut faire l'objet de nombreuses variantes de réalisation. De préférence, l'extrémité proximale 44 de la canule 42 est arrondie pour éviter de blesser les tissus ou organes déplacés, ainsi que les tissus ou organes environnants ceux qui déplacés par la palette 14. Par ailleurs, de préférence, la canule 40 est munie à son extrémité distale 46 de moyens de préhension tels que par exemple d'une collerette 48.

Le cas échéant, le tube 10 peut être placé dans une canule 40 possédant plusieurs lumières longitudinales séparées, les autres lumières étant susceptibles d'être utilisées à des fins connues en soi, telles que par exemple pour l'écoulement de fluide de traitement ou un prélèvement.

Lorsque la poire 22 occupe sa position de plus grande expansion, la palette gonflable 14 est affaissée et repliée longitudinalement le long des membrures 30, 31, 32. La palette 14 peut ainsi être logée en position rétractée à l'intérieur de la canule 40. Dans cette position, la palette 14 peut être amenée en tout point choisi approprié d'un corps à l'aide de la canule 40. Lorsque la palette 14 est amenée dans la zone d'utilisation, il suffit de déplacer le tube 10 à translation par rapport à la canule 40 et de comprimer la poire 22 pour gonfler la palette 14 dans sa position d'utilisation telle que représentée sur la figure 2.

Pour retirer l'instrument, il suffit ensuite de relacher la poire 22. La palette 14 reprend alors sa position repliée et peut être rétractée sans difficulté à l'intérieur de la canule 40.

L'instrument ainsi proposé dans le cadre de la présente invention, permet de déployer une palette 14 de grandes dimensions à travers une canule 40 de diamètre réduit. Par ailleurs, on notera que l'instrument ainsi proposé dans le cadre de la présente invention permet d'éviter en toute sécurité tout traumatisme des tissus ou organes déplacés, grâce à la souplesse de la palette 14 inhérente au matériau composant celle-ci, ainsi que tout traumatisme des tissus ou organes contactés par la canule 40 lors de l'introduction ou du retrait de l'instrument.

Le cas échéant un clapet anti-retour peut être prévu sur le tube 10 pour maintenir la palette 14 déployée sans exiger d'action continue sur la paire 22. Bien entendu ce clapet doit pouvoir être ouvert à la demande pour retracter la palette 14.

Par ailleurs la poire de gonflage/dégonflage 22 peut être remplacée par tout moyen équivalent, notamment par des moyens d'alimentation en fluide.

Comme schématisé sur la figure 3, la paroi transversale de liaison 17 peut être munie de deux lames symétriques 50, 52 qui se prolongent sur l'extrémité proximale du tube 10. Ces lames 50, 52 peuvent être réalisées en métal ou plastique. Elles permettent de renforcer l'instrument à l'égard d'efforts transversalement au plan des figures 2 et 3.

## Revendications

1. Instrument chirurgical, notamment pour chirurgie endoscopique, comprenant un tube (10), des moyens de gonflage/dégonflage (22) formés d'une poire (22) d'alimentation en fluide raccordée de façon étanche à l'extrémité distale (20) du tube (10) et une structure gonflable (14) placée à l'extrémité proximale du tube (10) et qui communique avec les moyens de gonflage/dégonflage (22) par l'intermédiaire du tube (10), caractérisé par le fait que la structure gonflable (14) est constituée d'une palette qui a la forme générale d'un éventail formé de deux toiles principales (15, 16) généralement parallèles entre elles à l'état gonflé, et raccordées sur leur périphérie par une paroi de liaison transversale (17) la palette gonflable (14) étant pourvue en outre de membrures de raidissement.

2. Instrument selon la revendication 1, caractérisé par le fait que le tube (10) est placé dans une canule (40).

3. Instrument selon l'une des revendications 1 ou 2, caractérisé par le fait que le tube (10), la palette gonflable (14) et la poire (22) sont réalisés sous forme d'une pièce unique.

4. Instrument selon l'une des revendications 1 ou 2, caractérisé par le fait que l'ensemble formé par le tube (10), la palette gonflable (14) et la poire (22) est réalisé par assemblage de pièces séparées.

5. Instrument selon l'une des revendications 1 à 4, caractérisé par le fait que la palette gonflable (14) est réalisée en caoutchouc.

6. Instrument selon l'une des revendications 1 à 5, caractérisé par le fait que les membrures de raidissement (30, 31, 32) sont venues de moulage avec la poche (14).

7. Instrument selon l'une des revendications 1 à 5, caractérisé par le fait que les membrures de raidissement (30, 31, 32) sont rapportées sur la poche gonflable (14).

8. Instrument selon l'une des revendications 1 à 7, caractérisé par le fait qu'un clapet anti-retour commandable est placé sur le tube (10).

## Claims

1. A surgical instrument, in particular for endoscopic surgery, the instrument comprising a tube (10), inflation/deflation means (22) constituted by a fluid feed bulb (22) connected in sealed manner to the distal end (20) of the tube (10) and an inflatable structure (14) placed at the proximal end of the tube (10) and which communicates with the inflation/deflation means (2) via the tube (10), the instrument being characterized by the fact that the inflatable structure (14) is constituted by a spatula which is generally fan-shaped and made from two main sheets (15, 16) which are generally parallel to each other in the inflated state and which are connected together at their peripheries by a transverse connecting wall (17), the inflatable spatula (14) being further provided with lines of stiffening.

2. An instrument according to claim 1, characterized by the fact that the tube (10) is placed in a cannula (40).

3. An instrument according to claim 1 or 2, characterized by the fact that the tube (10), the inflatable spatula (14), and the bulb (22) are made in the form of a single piece.

4. An instrument according to claim 1 or 2, characterized by the fact that the equipment constituted by the tube (10), the inflatable spatula (14), and the bulb (22) is made by assembling separate pieces.

5. An instrument according to any one of claims 1 to 4, characterized by the fact that the inflatable spatula (14) is made of rubber,

6. An instrument according to any one of claims 1 to 5, characterized by the fact that the lines of stiffening (30, 31, 32) are integrally molded with the spatula (14).

7. An instrument according to any one of claims 1 to 5, characterized by the fact that the lines of stiffening (30, 31, 32) are added to the inflatable spatula (14).

8. An instrument according to any one of claims 1 to 7, characterized by the fact that a controllable non-return valve is placed in the tube (10).

## Patentansprüche

1. Chirurgisches Instrument, insbesondere für die endoskopische Chirurgie, mit einer Röhre (10), Aufblas-/Ablaßmitteln (22), die aus einem dicht mit dem distalen Ende (20) der Röhre (10) verbundenen Ball (22) zur Zufuhr von Fluid gebildet sind, und einer aufblasbaren Konstruktion (14), die am proximalen Ende der Röhre (10) angeordnet und mittels der Röhre (10) mit den Aufblas-/Ablaßmitteln (22) verbunden ist, dadurch gekennzeichnet, daß die aufblasbare Konstruktion (14) aus einem Flügel besteht, der allgemein als Fächer ausgebildet ist, welcher aus zwei im aufgeblasenen Zustand allgemein zueinander parallelen Hauptsegeln (15, 16) gebildet ist, welche an ihrem Umfang durch eine Verbindungsquerwand (17) verbunden sind, wobei der aufblasbare Flügel (14) des weiteren mit Versteifungsrippen versehen ist.

2. Instrument nach Anspruch 1, dadurch gekennzeichnet, daß die Röhre (10) in einer Kanüle (40) plaziert ist.

3. Instrument nach Anspruch 1 oder 2, dadurch gekennzeichnet, daß die Röhre (10), der aufblasbare Flügel (14) und der Ball (22) einstückig hergestellt sind.

4. Instrument nach Anspruch 1 oder 2, dadurch gekennzeichnet, daß der aus der Röhre (10), dem aufblasbaren Flügel (14) und dem Ball (22) hergestellte Aufbau durch Zusammenbau einzelner Teile gebildet ist.

5. Instrument nach einem der Ansprüche 1 bis 4, dadurch gekennzeichnet, daß der aufblasbare Flügel (14) aus Gummi hergestellt ist.

6. Instrument nach einem der Ansprüche 1 bis 5, dadurch gekennzeichnet, daß die Versteifungsrippen (30, 31, 32) an dem Beutel (14) angeformt sind.

7. Instrument nach einem der Ansprüche 1 bis 5, dadurch gekennzeichnet, daß die Versteifungsrippen (30, 31, 32) an dem aufblasbaren Beutel (14) angefügt sind.

8. Instrument nach einem der Ansprüche 1 bis 7, dadurch gekennzeichnet, daß ein steuerbares Rückschlagventil an der Röhre (10) angeordnet ist.
